# DEMANDE DE BREVET EUROPEEN

(11) **EP 0 760 236 A1**
(43) Date de publication de la demande: **05.03.1997**
(21) Numéro de dépôt: 96401811.3
(22) Date de dépôt: 22.08.1996
(51) Int. Cl.: A61K 7/48, A61K 7/00, A61K 7/06

(54) **Mousse déformable à base d'huile, contenant un tensioactif fluoré**

(30) Priorité: 24.08.1995 FR 9510060
(71) Demandeur: L'OREAL, 75008 Paris (FR)
(72) Inventeur: Pastour, Valérie, 92150 Suresnes (FR); Pouget, Françoise, 94120 Fontenay sous Bois (FR); Fodor, Pierre, 92380 Garches (FR)
(74) Mandataire: Lhoste, Catherine

(57) **Abrégé**

La présente invention se rapporte à une mousse déformable stable du type émulsion, comprenant une phase huileuse dispersée dans une phase aqueuse à l'aide d'un émulsionnant, dans laquelle la phase huileuse représente au moins 75 % en poids de l'émulsion, la phase aqueuse renferme au plus 25 % en poids d'eau par rapport à l'émulsion et l'émulsionnant comprend au moins un émulsionnant fluoré.

Cette mousse est notamment destinée au traitement de la peau, y compris du cuir chevelu, des ongles et/ou des cheveux humains. Elle peut être aussi bien utilisée sur le visage que sur le corps par application topique.

## Description

La présente invention se rapporte à une émulsion se présentant sous forme d'une texture aérée stable ou mousse déformable, destinée notamment au traitement de la peau humaine, y compris du cuir chevelu, des ongles et/ou des cheveux. Cette mousse peut être aussi bien utilisée sur le visage que sur le corps humain par application topique.

Les utilisateurs de produits de soin de la peau ou de traitement dermatologique, recherchent de plus en plus des produits agréables à utiliser et ayant une texture originale. Jusqu'à présent, les compositions cosmétiques ou dermatologiques se présentent le plus souvent sous forme de solutions, de gels ou de crèmes plus ou moins fluides.

Classiquement, les crèmes sont constituées d'une émulsion. Les émulsions comprennent une phase aqueuse et une phase huileuse dispersées l'une dans l'autre. On recherche plus particulièrement les émulsions dont la phase externe est la phase aqueuse, car elles apportent plus de fraîcheur à l'application que les émulsions à phase externe huileuse. Leur toucher et leur application apparaissent moins gras que ceux d'une émulsion eau-dans-huile.

Jusqu'à ce jour, pour conférer aux émulsions une texture nouvelle, on y a introduit un gaz, généralement de l'air (voir notamment le document US-A-4755377) pour leur donner l'aspect d'une mousse. C'est ce que l'on appelle le foisonnement. Les émulsions aérées obtenues sont appréciées pour leur légèreté à l'application et pour leur dosage plus aisé que celui des crèmes classiques. Néanmoins, elles présentent l'inconvénient d'être relativement instables du fait de leur faible densité et donc de décanter après un certain temps de stockage. De plus, ces émulsions sont relativement fragiles et sont souvent déstabilisées quand on y ajoute des actifs cosmétiques et/ou dermatologiques.

Il subsiste donc le besoin d'une émulsion ayant l'aspect d'une mousse, qui reste stable dans le temps, même en présence d'actifs cosmétiques.

La demanderesse a trouvé de manière inattendue que l'on pouvait obtenir une mousse déformable en utilisant des émulsionnants fluorés déterminés dans une émulsion à phase continue aqueuse, contenant une forte proportion de phase huileuse et une faible quantité d'eau.

Aussi, l'invention a pour objet une mousse déformable du type émulsion, obtenue sans foisonnement, comprenant une phase huileuse dispersée dans une phase aqueuse à l'aide d'un émulsionnant, la phase huileuse représentant au moins 75 % en poids de l'émulsion, la phase aqueuse contenant de l'eau à au plus 25 % du poids total de l'émulsion, l'émulsionnant comprenant au moins un émulsionnant fluoré.

Selon l'invention, la phase aqueuse doit contenir au plus 25 % d'eau et de préférence au plus 20 % d'eau pour obtenir une mousse, le reste de la phase aqueuse contenant des adjuvants hydrophiles.

La mousse de l'invention présente une densité proche de celle d'une émulsion classique et elle reste très stable au cours du temps (plusieurs mois à température ambiante), ce qui n'est pas le cas des mousses actuellement disponibles sur le marché.

Personne jusqu'à ce jour n'avait fabriqué une mousse stable dans le temps (plusieurs mois à température ambiante), sans introduction de gaz, grâce à l'emploi d'un tensioactif fluoré et d'une faible quantité d'eau.

La mousse de l'invention est une émulsion huile-dans-eau qui constitue notamment une composition cosmétique et/ou dermatologique. A cet effet, elle contient, en outre, un ou plusieurs actifs cosmétiques et/ou dermatologiques.

Bien que contenant une quantité importante d'huile, cette mousse n'est ni grasse ni lourde et, comme la phase externe est la phase aqueuse, elle donne une impression de fraîcheur à l'application sur la peau malgré la faible quantité d'eau.

Certes, il est connu de préparer des émulsions huile-dans-eau comportant des émulsionnants fluorés. Ainsi, le document EP-A-641194 décrit des émulsions huile-dans-eau comprenant un perfluoropolyéther dans la phase huileuse, une phase aqueuse gélifiée et un émulsionnant fluoré. Cependant, cette émulsion se présente sous forme d'une crème classique et comporte une proportion importante d'eau (entre 70 et 80 %). En outre, ce document ne suggère pas que l'on puisse obtenir une texture mousse en augmentant la quantité de phase huileuse.

La mousse selon l'invention comporte avantageusement de 75 à 95 % en poids, et de préférence de 85 à 95 % en poids de phase huileuse par rapport au poids total de l'émulsion.

Dans la mousse selon l'invention, la phase huileuse comprend avantageusement au moins une huile hydrocarbonée notamment d'origine animale, végétale (par exemple l'huile de jojoba), minérale (par exemple l'huile de vaseline), ou synthétique (par exemple le palmitate d'éthyl hexyle, une huile siliconée (par exemple la diméthicone ou la cyclométhicone), et/ou une huile fluorée éventuellement siliconée. La phase huileuse contient avantageusement un mélange d'huiles. On peut citer notamment comme mélange d'huiles le mélange d'huile siliconée et de polyisobutène, vendu sous le nom « Fancorsil P » par la société Fancor.

De préférence, la phase huileuse renferme une huile fluorée associée de préférence à une huile hydrocarbonée. On peut citer notamment comme huile fluorée les perfluoropolyéthers, et notamment ceux vendus sous les dénominations « Fomblin » par la société Montedisson tels que le perfluorométhylisopropyléther vendu sous la dénomination Fomblin HC/25. Cette huile fluorée représente au plus 50 % de la phase huileuse. En jouant sur la proportion respective des huiles fluorées et hydrocarbonées, on peut modifier les qualités cosmétiques de la mousse, à savoir les propriétés nutritives, hydratantes, la douceur. En outre, la présence d'huile hydrocarbonée permet d'obtenir une composition à faible prix de revient.

L'émulsionnant fluoré représente avantageusement de 0,01 à 5 % en poids, de préférence de 0,01 à 2 % en poids par rapport au poids total de l'émulsion. On peut y ajouter un coémulsionnant non fluoré en une quantité allant avantageusement de 0,1 à 3 %, et de préférence de 0,5 à 2 % en poids, par rapport au poids total de l'émulsion. Il est tout à fait surprenant d'obtenir une mousse stable avec si peu de tensioactif. De plus l'avantage d'utiliser une si faible quantité de tensioactif permet de limiter les problèmes de tolérance (irritation, notamment de la peau).

De façon avantageuse, l'émulsionnant fluoré est un composé perfluoroalkyle. Par composé perfluoroalkyle, on entend un composé pouvant être partiellement ou totalement fluoré et pouvant contenir une ou plusieurs insaturations éthyléniques.

A titre d'émulsionnant fluoré, on peut utiliser selon l'invention les composés perfluoroalkyle de formule I à V suivantes :

(I) R_{f} - C₂H₄X

dans laquelle :
R_{f} représente une chaîne alkyle perfluorée, linéaire ou ramifiée, ayant de 4 à 20 atomes de carbone,
X représente un radical choisi parmi les radicaux suivants :
   (i) -COOY,
   (ii) -SO₃Y,
      Y représentant un atome d'hydrogène, un métal alcalin ou un groupe aminé tel qu'un groupe ammonium,
   (iii) -(OC₂H₄)ₘ-OH,
      m étant un nombre allant de 2 à 100, de préférence de 4 à 40,
   (iv) 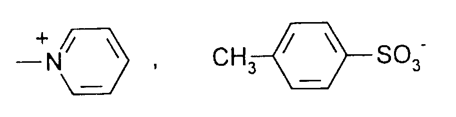
   (V) 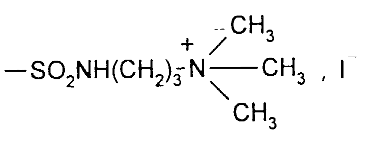
   (vi) 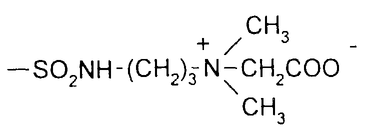 et
   (vii)

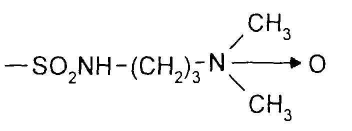

De préférence, le radical R_{f} des composés de formule (I) selon l'invention peut représenter une chaîne alkyle perfluorée, linéaire ou ramifiée, ayant de 4 à 6 atomes de carbone.

Comme composés de formule (I), on peut citer notamment ceux vendus sous les dénominations « Forafac » par la société Atochem, et notamment le Forafac 1179 de formule :

C₆F₁₃-C₂H₄-SO₂-NH-C₃H₆- N⁺(CH₃)₃l⁻

et le Forafac 1157 de formule :

C₆F₁₃-C₂H₄-SO₂-NH-C₃H₆- N⁺(CH₃)₂(CH₂COO⁻)

(II) R_{f}-R₁-R₂

dans laquelle :
R_{f} représente une chaîne alkyle perfluorée, linéaire ou ramifiée, ayant de 4 à 20 atomes de carbone,
R₁ est un reste SO₃, SO₂ ou CO₂ ;
R₂ est un reste choisi parmi :
   - NH₄⁺
   - N(R₃)CH₂-CO₂⁻X⁺
      R₃ étant un radical alkyle en C₁-C₄, et
      X est un atome d'hydrogène ou un métal alcalin,
   - NH(CH₂)ₚN⁺(R₃)₃I⁻
      p étant 1, 2, 3 ou 4 et
      R₃ étant tel que défini ci-dessus,
   - N(R₃)(CH₂CH₂O)-Y
      Y étant un atome d'hydrogène ou un radical alkyle en C₁-C₄, et
      R₃ étant tel que défini ci-dessus.

On peut en particulier citer comme composés de formule (II) ceux vendus sous les dénominations « Fluorad » par la société 3M.

(III) R_{f} - (CH₂)ₘ - S - Gₚ - H (lll)

dans laquelle :
R_{f} représente une chaîne alkyle perfluorée, linéaire ou ramifiée, ayant de 4 à 20 atomes de carbone,
m représente 0, 1 ou 2 ;
p représente une valeur statistique ou entière allant de 1 à 10 ;
G représente un motif choisi parmi :

   -CH₂-CH(O)-CH₂-O- , -CH-(CH₂-O)₂ , - CH₂-CH(OH)-CH₂-O-

   -CH(CH₂OH)- CH₂-O-, - CH₂-CH(CH₂OH)-O-

   chacun des atomes d'oxygène pouvant être relié à un atome d'hydrogène ou à un autre motif G.

Les composés de formule (III) sont décrits dans la demande de brevet FR-A-2694289.

(IV) R_{f} - (CH₂)ₘ-X-[(CHR)ᵣ-CH₂O]_{q}-H

dans laquelle :
R_{f} représente une chaîne alkyle perfluorée, linéaire ou ramifiée, ayant de 4 à 20 atomes de carbone,
m représente 0, 1 ou 2 ; de préférence, m = 2 ;
q est un nombre allant de 1 à 10 ; de préférence q = 10 ;
R est un groupe méthyle ou hydrogène ;
r = 1 quand R est le groupe méthyle et r = 1 ou 2 quand R = H ;
X est un atome d'oxygène ou de soufre.

On peut en particulier citer comme composés de formule (IV) ceux vendus sous les dénominations « Zonyl » par la société Du Pont, et notamment les « Zonyl FSN » et « Zonyl FSN 100 » de formule :

(CₙF₂ₙ₊₁) - CH₂CH₂O (CH₂CH₂O)ₓH avec n = 3 à 8,

le « Zonyl FSP » de formule :

[(CₙF₂ₙ₊₁) - (CH₂CH₂O)]_{1,2}P(O)(ONH₄)₂ avec n = 3 à 8.

(V) R_{f}-(CH₂)_{q}-O-CO-NR₁-CHR₂-(CH₂)ₚ-COOM

dans laquelle :
R_{f} représente une chaîne alkyle perfluorée, linéaire ou ramifiée, ayant de 4 à 20 atomes de carbone,
R₁ représente l'hydrogène ou une chaîne alkyle hydrocarbonée, linéaire ou ramifiée, ayant de 1 à 4 atomes de carbone,
R₂ représente l'hydrogène ou une chaîne alkyle hydrocarbonée, linéaire ou ramifiée, ayant de 1 à 4 atomes de carbone, ou un groupement
- (CH₂)ₘ - COOH avec m égal à 1 ou 2, ou un groupement -CH₂OH, ou un groupement -CHOH-CH₃,
M représente l'hydrogène ou un cation organique ou inorganique,
q est un nombre allant de 0 à 4, et
p est 0 ou 1.

De préférence, le radical R_{f} des composés de formule (V) selon l'invention peut représenter une chaîne alkyle perfluorée, linéaire ou ramifiée, ayant de 6 à 8 atomes de carbone, le radical R₁ peut représenter une chaîne alkyle hydrocarbonée linéaire ou ramifiée ayant de 1 à 4 atomes de carbone, et le radical R₂ peut représenter une chaîne alkyle hydrocarbonée linéaire ou ramifiée ayant de 1 à 4 atomes de carbone.

En particulier, quand p = 0, R₂ peut être l'hydrogène ou -(CH₂)₂COOH. Quand p=1, R₂ peut être l'hydrogène.

Parmi les composés de formule (V), on peut citer :
. la N-2-F-octyl éthyloxycarbonyl-β-alanine,
. la N-2-F-octyl éthyloxycarbonyl-sarcosine,
. la N-2-F-hexyl éthyloxycarbonyl-sarcosine,
. l'acide N-2-F-octyl éthyloxycarbonyl-L-glutamique,
. l'acide N-2-F-hexyl éthyloxycarbonyl-L-glutamique,
. le sel de sodium de la N-2-F-octyl éthyloxycarbonyl-sarcosine,
. le sel de triéthanolamine de la N-2-F-octyl éthyloxycarbonyl-sarcosine,
. le sel de sodium de l'acide N-2-F-octyl éthyloxycarbonyl-L-glutamique, et
. le sel de triéthanolamine de l'acide N-2-F-octyl éthyloxycarbonyl-L-glutamique.

Parmi les composés de formule (V), on utilise de préférence dans l'émulsion selon l'invention le sel de sodium de la N-2-F-octyl éthyloxycarbonyl-sarcosine.

Les composés de formule (V) sont préparés par un procédé qui consiste à faire réagir, de préférence en milieu eau-solvant organique, un composé de formule :

R_{f}-(CH₂)ₘ - O - CO - X ,

avec un composé de formule :

NHR₁ - CHR₂ - (CH₂)ₚ - COO⁻ Y⁺,

dans lesquelles R_{f} , R₁, R₂ , m et p ont les mêmes significations que précédemment, X est choisi dans le groupe constitué par un atome de chlore, un radical chlorométhyle, un radical azolyle et de préférence un radical imidazolyle, et Y est choisi parmi les cations organiques ou inorganiques, de préférence le sodium ou le potassium.

Le coémulsionnant non fluoré utilisable dans l'émulsion selon l'invention peut être notamment un coémulsionnant hydrocarboné. Comme coémulsionnant hydrocarboné, on peut citer par exemple les esters d'acides gras polyoxyéthylénés et/ou polyoxypropylénés et en particulier les Polysorbates tels que le Polysorbate 80. Le coémulsionnant est de préférence présent en une quantité allant de 0,1 à 5 % en poids, et de préférence de 0,5 à 1 % en poids par rapport au poids total de l'émulsion.

La mousse selon l'invention trouve son application dans un grand nombre de traitements cosmétiques et/ou dermatologiques de la peau humaine, y compris le cuir chevelu et les ongles, comme par exemple l'hydratation, la nutrition, la protection, la dépigmentation, le raffermissement, le traitement du vieillissement de la peau humaine, le traitement des rides et/ou des ridules du visage et/ou du corps humain, l'amincissement du corps humain, le traitement de l'acné, et les traitements des maladies de la peau humaine telles que les mycoses, les dermites et le psoriasis.

Aussi, l'invention a encore pour objet une utilisation de la mousse définie ci-dessus pour le traitement cosmétique de la peau humaine, du visage et/ou du corps, des cheveux et des ongles, notamment pour hydrater, nourrir, protéger, raffermir, éliminer les rides et/ou les ridules et/ou l'acné, amincir et/ou dépigmenter, ainsi que pour la préparation d'une composition destinée au traitement des maladies de la peau et/ou des ongles, et notamment des mycoses, des dermites et du psoriasis.

L'invention a encore pour objet un procédé de traitement cosmétique et/ou dermatologique de la peau, des ongles et/ou des cheveux consistant à appliquer la mousse ci-dessus sur la peau, les ongles et/ou les cheveux.

De façon connue, la mousse de l'invention peut, en outre, contenir des adjuvants habituels dans les domaines cosmétique ou dermatologique, tels que les conservateurs, les antioxydants, les parfums, les charges, les filtres, les matières colorantes, les actifs hydrophiles ou lipophiles, et encore des vésicules lipidiques. Ces adjuvants sont utilisés dans les quantités habituelles dans les domaines considérés et par exemple de 0,1 à 10 % en poids par rapport au poids total de l'émulsion.

Les actifs cosmétiques ou dermatologiques pour la peau peuvent être des actifs anti-âge, des actifs anti-rides, des hydratants ou des humectants, des actifs amincissants, des actifs dépigmentants, des actifs anti-radicaux libres, des actifs nutritifs, des actifs protecteurs, des actifs restructurants, des actifs raffermissants, des actifs antiacnéiques, des actifs exfoliants, des actifs émollients ou encore des actifs traitant les maladies de peau comme les mycoses, les dermites, le psoriasis, etc. Ces actifs sont utilisés, selon leur nature, dans les proportions habituelles dans les domaines considérés, et par exemple de 0,01 % à 10 % en poids par rapport au poids total de l'émulsion.

On donne ci-après des exemples d'émulsions selon l'invention à titre illustratif et non limitatif. Toutes les émulsions sont préparées par mélange de la phase aqueuse et des tensioactis à froid sous agitation à l'homogénéisateur, puis incorporation de l'huile peu à peu. Les pourcentages des compositions sont des pourcentages en poids.

### Exemple 1 : Préparation du sel de sodium de la N-2-F-octyl éthyloxycarbonyl-sarcosine

On solubilise 115,8 g de N-2-F-octyl éthyloxycarbonyl-sarcosine dans 40 ml d'éthanol. 8 g de soude en pastille sont solubilisés dans 1000 ml d'éthanol. La solution de soude est ajoutée dans la première solution. Il se forme un précipité blanc. Le mélange est agité pendant 1 heure, filtré et séché. On obtient 94,6 g d'une poudre blanche (rendement 78%).

L'analyse chimique du produit obtenu donne les résultats suivants :
- Température de fusion : 170°C
- Analyse élémentaire :

| | % C | % H | % N | % F | % Na |
|---|---|---|---|---|---|
| Théorique | 27,97 | 1,51 | 2,33 | 53,72 | 3,82 |
| Expérimental | 27,20 | 1,72 | 2,46 | 52,32 | 3,86 |

### Exemple 2 : Produit de soin pour le visage

| | |
|---|---|
| Zonyl FSN (Emulsionnant fluoré à 18 % en matière active) | 2,2 % |
| Perfluorométhylisopropyléther (Fomblin HC/25) | 45,55 % |
| Huile de vaseline | 45,55 % |
| Eau | QSP 100 % |

On obtient une mousse présentant une texture légère, qui pénètre bien dans la peau et laisse la peau douce sans la graisser malgré les 92,9 % d'huiles.

### Exemple 3 : Produit pour le corps

| | |
|---|---|
| Forafac 1157 (Emulsionnant fluoré à 26,7 % en matière active) | 2,8 % |
| Perfluorométhylisopropyléther (Fomblin HC/25) | 1 % |
| Polysorbate 80 (Coémulsionnant) | 0,75 % |
| Palmitate d'éthyl hexyle | 15 % |
| Cyclométhicone | 39,5 % |
| Fancorsil P | 36 % |
| Eau | qsp 100 % |

On obtient une mousse fluide pour le corps, présentant une texture aérée, qui s'étale bien et n'est pas gras à l'application.

### Exemple 4 : Produit pour les mains

| | |
|---|---|
| Huile de jojoba | 15 % |
| Diméthicone (0,65 cst) | 67,5 % |
| Sel de sodium de la N-2-F-octyl éthyloxycarbonyl-sarcosine (Emulsionnant fluoré) | 0,4 % |
| Eau | qsp 100 % |

On obtient une mousse pour les mains de texture aérée, agréable à utiliser et qui pénètre bien.

### Exemple 5 : Produit de soin

| | |
|---|---|
| Huile de vaseline | 46,45 % |
| Perfluorométhylisopropyléther (Fomblin HC/25) | 46,45 % |
| Sel de sodium de la N-2-F-octyl éthyloxycarbonyl-sarcosine (Emulsionnant fluoré) | 0,4 % |
| Eau | 6,7 % |

On obtient un produit de soin de texture aérée, agréable à utiliser sur le visage ou le corps.

### Exemple 6 : Produit de soin

| | |
|---|---|
| Huile de vaseline | 39,8 % |
| Perfluorométhylisopropyléther (Fomblin HC/25) | 39,8 % |
| Sel de sodium de la N-2-F-octyl éthyloxycarbonyl-sarcosine (Emulsionnant fluoré) | 0,4 % |
| Eau | 20 % |

L'émulsion obtenue se présente sous forme d'une mousse stable, pénétrant bien et non grasse.

### Exemple 7 : Produit de soin

| | |
|---|---|
| Huile de vaseline | 45,6 % |
| Perfluorométhylisopropyléther (Fomblin HC/25) | 45,6 % |
| Zonyl FSP (Emulsionnant fluoré à 18 % en matière active) | 2,2 % |
| Eau | 6,6 % |

On obtient une émulsion homogène et fine, ayant la texture mousse légère, agréable à utiliser.

### Exemple 8 : Produit de soin

| | |
|---|---|
| Huile de vaseline | 45,6 % |
| Perfluorométhylisopropyléther (Fomblin HC/25) | 45,6 % |
| Zonyl FSN (Emulsionnant fluoré à 18 % en matière active) | 2,2 % |
| Eau | 6,6 % |

L'émulsion obtenue se présente sous forme d'une mousse légère ayant une bonne stabilité.

## Revendications

1. Mousse déformable du type émulsion, obtenue sans foisonnement, comprenant une phase huileuse dispersée dans une phase aqueuse à l'aide d'un émulsionnant, la phase huileuse représentant au moins 75 % en poids de l'émulsion, la phase aqueuse contenant de l'eau à au plus 25 % du poids total de l'émulsion, l'émulsionnant comprenant au moins un émulsionnant fluoré.

2. Mousse selon la revendication 1, caractérisée en ce en ce que la phase huileuse représente de 75 à 95 % en poids par rapport au poids total de l'émulsion.

3. Mousse selon la revendication 2, caractérisée en ce que la phase huileuse représente de 85 à 95 % en poids par rapport au poids total de l'émulsion.

4. Mousse selon l'une quelconque des revendications précédentes, caractérisée en ce qu'elle contient de 0,01 à 5 % en poids d'émulsionnant fluoré par rapport au poids total de l'émulsion.

5. Mousse selon l'une quelconque des revendications précédentes, caractérisée en ce qu'elle contient de 0,01 à 2 % en poids d'émulsionnant fluoré par rapport au poids total de l'émulsion.

6. Mousse selon l'une quelconque des revendications précédentes, caractérisée en ce que la phase huileuse contient une huile fluorée.

7. Mousse selon l'une quelconque des revendications précédentes, caractérisée en ce que la phase huileuse contient au plus 50 % d'une huile fluorée.

8. Mousse selon l'une quelconque des revendications précédentes, caractérisée en ce que la phase huileuse contient une huile fluorée et une huile hydrocarbonée.

9. Mousse selon l'un quelconque des revendications précédentes, caractérisée en ce qu'elle contient au moins un actif cosmétique et/ou dermatologique.

10. Mousse selon l'une quelconque des revendications précédentes, caractérisée en ce que l'émulsionnant fluoré est un composé perfluoroalkyle.

11. Mousse selon l'une quelconque des revendications précédentes, caractérisée en ce que l'émulsionnant fluoré est un composé perfluoroalkyle de formule :
(I) R_{f} - C₂H₄X
dans laquelle :
R_{f} représente une chaîne alkyle perfluorée, linéaire ou ramifiée, ayant de 4 à 20 atomes de carbone,
X représente un radical choisi parmi les radicaux suivants :
(i) -COOY,
(ii) -SO₃Y,
Y représentant un atome d'hydrogène, un métal alcalin ou un groupe aminé tel qu'un groupe ammonium,
(iii) - (OC₂H₄)ₘ-OH,
m étant un nombre allant de 2 à 100, de préférence de 4 à 40,
(iv) 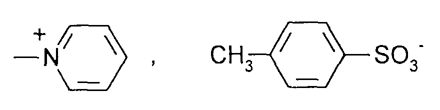
(v) 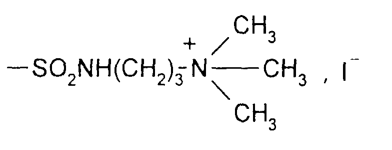
(vi) 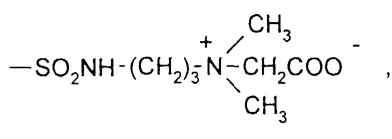 et
(vii)

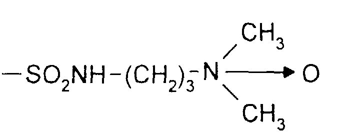

12. Mousse selon l'une quelconque des revendications 1 à 10, caractérisée en ce que l'émulsionnant fluoré est un composé perfluoroalkyle de formule :
(II) R_{f} - R₁ - R₂
dans laquelle :
R_{f} représente une chaîne alkyle perfluorée, linéaire ou ramifiée, ayant de 4 à 20 atomes de carbone,
R₁ est un reste SO₃, SO₂ ou CO₂ ;
R₂ est un reste choisi parmi :
- NH₄⁺
- N(R₃)CH₂-CO₂ ⁻ X⁺
R₃ étant un radical alkyle en C₁-C₄, et
X est un atome d'hydrogène ou un métal alcalin,
- NH(CH₂)ₚN⁺(R₃)₃ l⁻
p étant 1, 2, 3 ou 4 et
R₃ étant tel que défini ci-dessus,
- N(R₃)(CH₂CH₂O)-Y
Y étant un atome d'hydrogène ou un radical alkyle en C₁-C₄, et
R₃ étant tel que défini ci-dessus.

13. Mousse selon l'une quelconque des revendications 1 à 10, caractérisée en ce que l'émulsionnant fluoré est un composé perfluoroalkyle de formule :
(III) R_{f} - (CH₂)ₘ - S - Gₚ - H (III)
dans laquelle :
R_{f} représente une chaîne alkyle perfluorée, linéaire ou ramifiée, ayant de 4 à 20 atomes de carbone,
m représente 0, 1 ou 2 ;
p représente une valeur statistique ou entière comprise entre 1 et 10 ;
G représente un motif choisi parmi :
-CH₂-CH(O)-CH₂-O- , -CH-(CH₂-O)₂ , - CH₂-CH(OH)-CH₂-O-
-CH(CH₂OH)-CH₂-O-, -CH₂-CH(CH₂OH)-O-
chacun des atomes d'oxygène pouvant être relié à un atome d'hydrogène ou à un autre motif G.

14. Mousse selon l'une quelconque des revendications 1 à 10, caractérisée en ce que l'émulsionnant fluoré est un composé perfluoroalkyle de formule :
(IV) R_{f} - (CH₂)ₘ-X-[(CHR)ᵣ-CH₂O]_{q}-H
dans laquelle :
R_{f} représente une chaîne alkyle perfluorée, linéaire ou ramifiée, ayant de 4 à 20 atomes de carbone,
m représente 0, 1 ou 2 ; de préférence, m = 2 ;
q est un nombre allant de 1 à 10 ; de préférence q = 10 ;
R est un groupe méthyle ou hydrogène ;
r = 1 quand R est le groupe méthyle et r = 1 ou 2 quand R = H ;
X est un atome d'oxygène ou de soufre.

15. Mousse selon l'une quelconque des revendications 1 à 10, caractérisée en ce que l'émulsionnant fluoré est un composé perfluoroalkyle de formule :
(V) R_{f}-(CH₂)_{q}-O-CO-NR₁-CHR₂-(CH₂)ₚ-COOM
dans laquelle :
R_{f} représente une chaîne alkyle perfluorée, linéaire ou ramifiée, ayant de 4 à 20 atomes de carbone,
R₁ représente l'hydrogène ou une chaîne alkyle hydrocarbonée, linéaire ou ramifiée, ayant de 1 à 4 atomes de carbone,
R₂ représente l'hydrogène, ou une chaîne alkyle hydrocarbonée, linéaire ou ramifiée, ayant de 1 à 4 atomes de carbone, ou un groupement
- (CH₂)ₘ - COOH avec m égal à 1 ou 2, ou un groupement -CH₂OH, ou un groupement -CHOH-CH₃,
M représente l'hydrogène ou un cation organique ou inorganique,
q est un nombre allant de 0 à 4, et
p est 0 ou 1.

16. Mousse selon l'une quelconque des revendications précédentes, caractérisée en ce que l'émulsion contient en outre un coémulsionnant.

17. Mousse selon l'une quelconque des revendications précédentes, caractérisée en ce qu'elle contient de 0,1 à 5 % en poids de coémulsionnant par rapport au poids total de l'émulsion.

18. Mousse selon l'une quelconque des revendications précédentes, caractérisée en ce qu'elle constitue une composition cosmétique et/ou dermatologique.

19. Utilisation de la mousse selon l'une quelconque des revendications précédentes pour un traitement cosmétique de la peau, des ongles et/ou des cheveux humains.

20. Utilisation de la mousse selon l'une quelconque des revendications 1 à 18 pour hydrater, nourrir, protéger, raffermir et/ou dépigmenter la peau humaine, et/ou éliminer les rides et/ou les ridules du visage et/ou du corps humain et/ou amincir le corps humain.

21. Utilisation de la mousse selon l'une quelconque des revendications 1 à 18 pour la préparation d'une composition destinée au traitement des maladies de la peau et/ou des ongles humains.

22. Procédé de traitement cosmétique de la peau, des ongles et/ou des cheveux humains, caractérisé en ce qu'il consiste à appliquer sur la peau, les ongles et/ou les cheveux une mousse selon l'une quelconque des revendications 1 à 18.
